# EUROPEAN PATENT SPECIFICATION

(11) **EP 1 906 830 B1**
(45) Date of publication and mention of the grant of the patent: **04.09.2013**
(21) Application number: 06766160.3
(22) Date of filing: 20.07.2006
(51) Int. Cl.: A61F 2/02, A61F 2/01, A61H 21/00, A61H 23/02, A61F 5/00

(54) **EXTENDING INTRABODY CAPSULE**
VERLÄNGERNDE INTRAKÖRPER-KAPSEL
CAPSULE INTRACORPORELLE EXTENSIBLE

(30) Priority: 26.07.2005 US 702782 P
(43) Date of publication of application: 09.04.2008
(73) Proprietor: Weiss, Ram, 34485 Haifa (IL); Weiss, Menachem P., 34485 Haifa (IL)
(72) Inventor: Weiss, Ram, 34485 Haifa (IL); Weiss, Menachem P., 34485 Haifa (IL)
(74) Representative: Freeman, Avi
(86) International application number: PCT/IL2006/000845
(87) International publication number: WO 2007/013059

(56) References cited:
- US-A- 5 733 313
- US-A- 5 868 141
- US-A1- 2002 183 783
- US-A1- 2003 216 622
- US-A1- 2004 186 503

## Description

### FIELD OF THE INVENTION

The present invention relates generally to medical devices, and particularly to intrabody capsules.

### BACKGROUND OF THE INVENTION

Several methods are known in the art for treating medical conditions using devices inserted into the patient's body. For example, U.S. Patent 4,607,618, describes a method for treatment of morbid obesity. A hollow-shaped appliance is placed in a patient stomach. The appliance is formed of semi-rigid hollow-shaped appliance is placed in a patient stomach. The appliance is formed of semi-rigid skeleton members and is collapsible to a dimension and shape which can be inserted into the stomach through the esophagus and cardiac opening. Upon release of the collapsed device in the stomach, it autogenously re-assumes its normal uncollapsed shape.

U.S. Patent 4,648,383, describes an apparatus for treatment of morbid obesity. The apparatus includes a collapsible intra-gastric appliance, which can be temporarily deformed to pass through the esophagus and cardiac opening of the stomach and to autogenously assume a normal shape after it is received in the stomach to stimulate neuro-receptors in the sub-mucosa of the gastric fundus. Means are provided for detachably connecting the appliance to the lower end of an elongate, semi-rigid inserter rod which is passed through an aperture formed in the appliance to effect the detachable connection. Downward pressure on the inserter rod forces the collapsed appliance through the esophagus and cardiac opening into the stomach and slight upward force of the inserter rod is thereafter applied to detach the rod from the appliance.

Other methods for treating obesity involve the insertion of an elastic expandable device, such as an inflatable balloon, into the patient's stomach. Such methods are described, for example, in U.S. Patents 5,993,473, 6,454,785, 4,246,893, 4,485,805, 4,315,509, 4,416,267 and 6,733,512.

Other methods for treating obesity involve the use of electric signals and currents applied to the stomach. Such methods are described, for example, in U.S. Patent Application Publications 2004/0122453, 2005/0245957 and 2005/0183732. US 2003/0216622 discloses a capsule for insertion into the gastro-intestinal tract, comprising arms arranged to expand from the capsule body in order to anchor the capsule at a location. A disassembly mechanism is arranged for disengaging the arms from the capsule body.

### SUMMARY OF THE INVENTION

Embodiments of the present invention provide improved methods and systems for treating various medical conditions using intrabody devices inserted into a patient's body. The embodiments described herein mainly address devices inserted into the gastrointestinal tract, specifically the stomach, in order to reduce caloric intake, although similar intrabody objects can be used to treat other tubular or hollow organs and other conditions, as well.

In some embodiments, a capsule is inserted orally into the patient's stomach. The capsule comprises a capsule body and one or more self-extending arms attached to the body. During insertion, the arms are wrapped around the capsule body or otherwise tightly packed against or within the capsule body, so as to enable smooth and safe passage via the esophagus. When the capsule reaches the desired location in the stomach, the self-extending arms are released and allowed to extend. When extended, the arms come into contact with multiple points on the inner surface of the stomach.

The arms may be driven by the capsule to change their position and/or shape in response to electrical signals generated by the capsule. For example, the arms may comprise a piezoelectric material, to which the capsule applies a varying electrical potential. The arms may thus perform a dual role: On one hand, the arms anchor the capsule at the desired location. The arms apply a mechanical, tactile and/or nociceptive stimulation to the tissue in its vicinity, in this case - the stomach. This stimulation induces hormonal and neuronal stimulatory effects that suppress the patient's appetite and reduce caloric intake by several possible mechanisms. The mechanical stimulation is enhanced by vibrating the anchored capsule.

In some embodiments, the intrabody capsules described herein can generally be used to alter and control the caloric intake of the patient, either constantly or on a time-varying basis. As will be shown below, the capsule alters the mucosal metabolic profile, i.e., affects the metabolism, of the tissue it contacts. Moreover, the local stimulation may cause a specific profile of neuronal discharge from the target organ to the central nervous system, thus inducing systemic effects.

The capsule is controlled by an external operator console, which transmits commands to a receiver in the capsule. For example, the capsule can be activated and instructed to expand using an external command. The capsule comprises a computerized customizable controller that can be activated by the external commands to perform preprogrammed functions and additional functions at will. When desired, the capsule can be instructed to disassemble. A disassembly mechanism separates the arms from the capsule body, and the different capsule elements are naturally disposed of via the patient's digestive system. All elements of the device are thus able to advance passively, distal from its location of activity. In other organs, the elements of the device may be evacuated naturally or by surgical means, depending on the organ or system involved.

Several exemplary configurations of self-extending capsules are described hereinbelow. Additionally or alternatively to applying tissue stimulation, in some embodiments the capsules perform additional functions. For example, a capsule may perform localized administration of medications and other substances.

There is therefore provided, in accordance with an embodiment of the present invention, a device for insertion into an organ of a patient, including the features of claim 1.

In some embodiments, the organ includes a part of a gastrointestinal tract, a genitourinary tract, a blood vessel, a part of a central nervous system, an intra-abdominal organ, a thoracic organ or a limb of the patient.

In an embodiment, the device is arranged to be swallowed by the patient. Alternatively, the device may be arranged to be inserted into the organ using an endoscope. Further alternatively, the device may be arranged to be inserted into the organ using invasive means.

In another embodiment, the device has a diameter not exceeding 15 millimeters prior to self-extension of the one or more arms. The arms typically include at least one of a metal, a plastic and a piezoelectric material.

In yet another embodiment, the device includes an antenna and a receiver, which are arranged to receive external commands transmitted to the device from an external transmitter, and a controller, which is arranged to control an operation of the device responsively to the external commands. In still another embodiment, the controller is arranged to cause the one or more arms to extend from the capsule body responsively to at least one of the external commands and a pre-programmed schedule. Additionally or alternatively, the controller is arranged to cause one or more arms to disengage from the capsule body responsively to at least one of the external commands and a pre-programmed schedule.

In an embodiment, the device includes a release mechanism, which is arranged to restrain the one or more arms during the insertion into the organ, and a controller, which is arranged to actuate the release mechanism to release the one or more arms, so as to permit self-expansion of the arms at the desired location in the organ.

In another embodiment, the one or more arms are elastic and arranged to assume an extended position when not restrained, and the one or more arms are wrapped around the capsule body during the insertion into the organ. The one or more arms may include respective pivots connecting the arms to the capsule body, and the one or more arms may be arranged to rotate around the respective pivots when expanding.

In some embodiments, the one or more arms include a piezoelectric material, and the controller is arranged to apply a voltage to the piezoelectric material so as to cause the one or more arms to extend.

In an embodiment, the device includes a controller, which is arranged to determine a release condition and to actuate the release mechanism responsively to the release condition so as to cause the release mechanism to release the one or more arms. In another embodiment, the release condition includes at least one of a predetermined time schedule and an external command transmitted to the device.

In another embodiment, the release mechanism includes a shape memory alloy (SMA) element, and the controller is arranged to actuate the release mechanism using the SMA element. Additionally or alternatively, the release mechanism includes a motor, and the controller is arranged to actuate the release mechanism using the motor. Further additionally or alternatively, the release mechanism includes a piezoelectric element, and the controller is arranged to actuate the release mechanism using the piezoelectric element. In yet another embodiment, the release mechanism includes a solenoid, and the controller is arranged to actuate the release mechanism using the solenoid. In still another embodiment, the release mechanism includes a detent, and the controller is arranged to cause a removal of the detent so as to actuate the release mechanism.

The device includes a disassembly mechanism, which is arranged to disengage the one or more arms from the capsule body so as to allow the capsule body and the one or more arms to be disposed of.

In an embodiment, the one or more arms include respective pivots connecting the one or more arms to the capsule body, and the disassembly mechanism is arranged to remove the pivots.

In another embodiment, the device includes a controller, which is arranged to actuate the disassembly mechanism responsively to a disassembly condition. The disassembly condition may include a predetermined time schedule, a low power condition of a power source of the device or an external command transmitted to the device.

In yet another embodiment, the disassembly mechanism includes a shape memory alloy (SMA) element, and the controller is arranged to actuate the disassembly mechanism using the SMA element. In still another embodiment, the disassembly mechanism includes a motor, and the controller is arranged to actuate the disassembly mechanism using the motor. In another embodiment, the disassembly mechanism includes a piezoelectric element, and the controller is arranged to actuate the disassembly mechanism using the piezoelectric element. In yet another embodiment, the disassembly mechanism includes a solenoid, and the controller is arranged to actuate the disassembly mechanism using the solenoid. Additionally or alternatively, the disassembly mechanism includes a ball lock mechanism. Further additionally or alternatively, the disassembly mechanism includes a detent, and the controller is arranged to cause a removal of the detent so as to actuate the disassembly mechanism.

When extended, the one or more arms are arranged to apply at least one of a mechanical, a tactile and a nociceptive stimulation at respective contact points in the organ. The one or more arms may be arranged to transfer a mechanical vibration to the respective contact points.

The device includes a resonator attached to the capsule body, which is arranged to vibrate the one or more arms. The resonator may be arranged to vibrate the one or more arms with a random vibration having frequencies within a narrow bandwidth containing respective resonant frequencies of the one or more arms.

In another embodiment, the one or more arms include piezoelectric material, and the device includes a controller, which is arranged to drive the one or more arms with respective electrical signals so as to cause the one or more arms to vibrate. In yet another embodiment, the electrical signals have frequencies that approximate respective resonant frequencies of the one or more arms.

In still another embodiment, the one or more arms include respective feedback elements, which are arranged to sense vibrations of the arms, and the controller is arranged to adjust respective frequencies of the vibrations of the arms to match resonant frequencies of the arms based on the vibrations sensed by the feedback elements.

In some embodiments, the device is arranged to discharge a substance at the desired location within the organ. The device may include a container holding the substance fitted in the capsule body. Additionally or alternatively, the substance may be impregnated in at least one of an outer surface of the capsule body and the one or more arms.

In an embodiment, the device includes a transmitter, which is arranged to transmit a status of the device to an external receiver.

In another embodiment, the arms include a piezoelectric material, and the device includes a controller, which is arranged to expand and contract the arms by applying electrical voltages to the piezoelectric materials so as to enable the device to move within the organ. The controller may be arranged to control a motion of each of the one or more arms separately so as to navigate the device within the organ. In yet another embodiment, the device includes a camera, which is arranged to produce images of the organ, and the controller is arranged to automatically identify a pattern in the images and to navigate the device responsively to the identified pattern.

In some embodiments, at least one of the capsule body and the one or more arms include a material that provides a high contrast when imaged by a medical imaging system. In an embodiment, the one or more arms have curved a-traumatic distal ends. Additionally or alternatively, the one or more arms have curved profiles so as to increase a structural rigidity of the arms.

In another embodiment, the device includes a power source including at least one of a battery, a rechargeable battery and a radio frequency (RF) coil arranged to accept externally-transmitted RF energy.

### BRIEF DESCRIPTION OF THE DRAWINGS

Fig. 1 is a schematic, pictorial illustration of a system for treating obesity, in accordance with an embodiment of the present invention;
Fig. 2 is a block diagram that schematically illustrates functional components of an intrabody capsule, in accordance with an embodiment of the present invention;
Figs. 3A, 3B, 4A and 4B are longitudinal and transverse cross-sections of intrabody capsules, in accordance with embodiments of the present invention;
Figs. 5A-5E are schematic illustrations of self-extracting arms, in accordance with embodiments of the present invention;
Fig. 6 is a longitudinal cross-section of an intrabody capsule, in accordance with yet another embodiment of the present invention;
Figs. 7A-7C are schematic illustrations of a self-extracting and vibrating arm, in accordance with an embodiment of the present invention;
Figs. 8A and 8B are transverse cross-sections of intrabody capsules, in accordance with embodiments of the present invention;
Figs. 9A and 9B are cross-sections of lock and release mechanisms of self- extracting arms, in accordance with embodiments of the present invention;
Fig. 10 is a longitudinal cross-section of an intrabody capsule, in accordance with an alternative embodiment of the present invention; and
Figs. 11A and 11B are schematic illustration of anchoring of various capsules within an intrabody cavity, in accordance with an embodiment of the present invention.

### DETAILED DESCRIPTION OF EMBODIMENTS

### SYSTEM DESCRIPTION

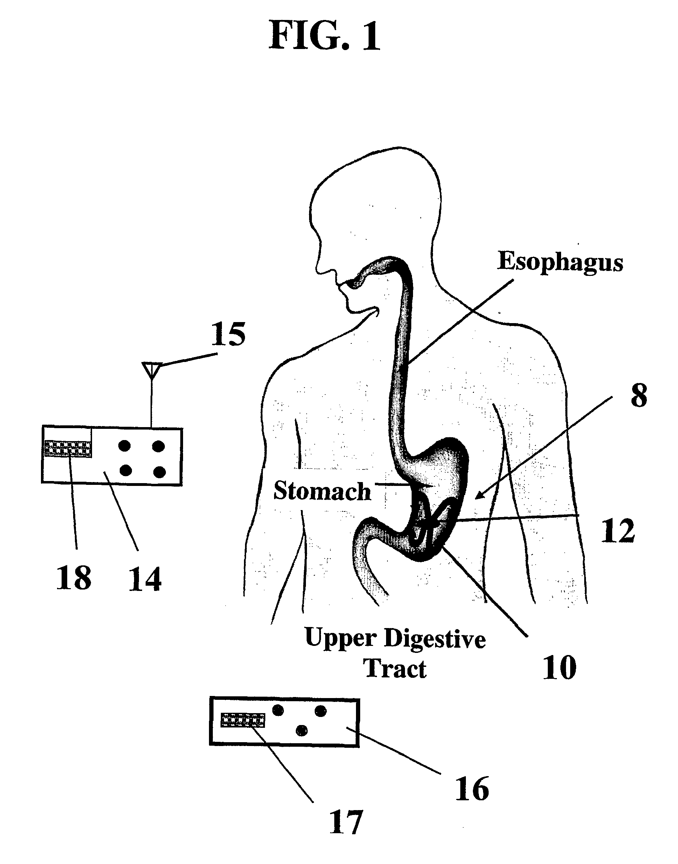

Fig. 1 is a schematic, pictorial illustration of a system for treating obesity, in accordance with an embodiment of the present invention. A miniature capsule 8 is swallowed or inserted orally via the mouth and the esophagus, into the patient's stomach. The capsule may either be swallowed by the patient or be inserted using an endoscope.

The capsule comprises a capsule body 12 and one or more self-extending arms 10. The different capsule elements are typically bio-compatible and a-traumatic. During insertion of the capsule, arms 10 are folded, wrapped, collapsed or otherwise tightly packed adjacently to the capsule body, in order to enable the capsule to safely and conveniently pass through the esophagus. At a desired location within the patient's body, such as upon reaching a particular location in the stomach, the arms self-extract to their expanded form. Typically, the capsule comprises multiple arms, which extract and contact the inner surface of the stomach at multiple points, thus anchoring the capsule at the desired location and preventing it from advancing distally within the lumen.

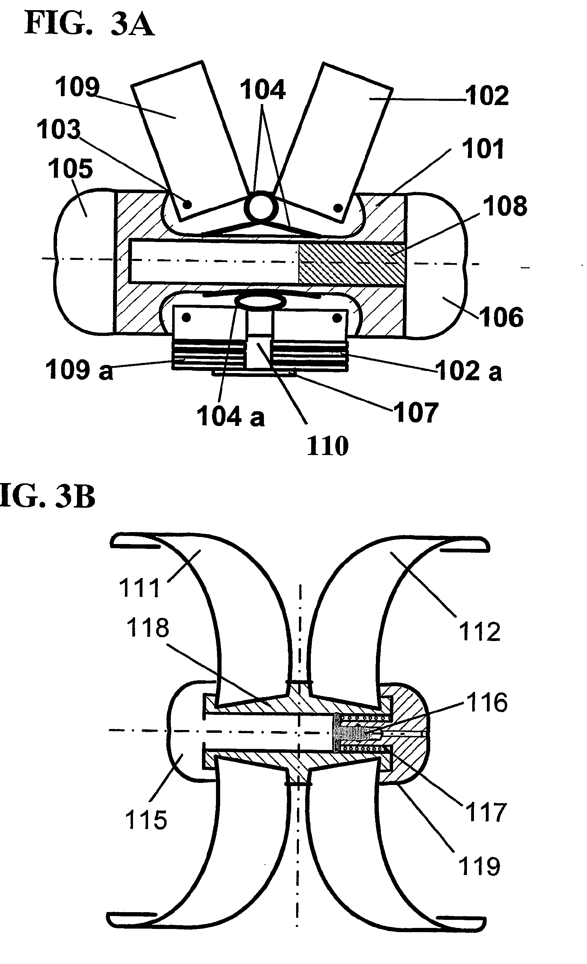

When swallowed or inserted, the capsule is tightly packed into a cylindrical or oval shape, so as to enable easy and safe insertion via the patient's esophagus. In its packed form, the capsule has a typical diameter of 8-12 mm and a typical length of 15-23 mm. When extended, each arm has a typical span of up to 60 mm. The dimensions indicated are given by way of example, and any other suitable dimensions can be used, depending on the organ and location of interest. The capsule dimensions may vary depending on its contents and configuration. Several exemplary capsule configurations are described in detail in Figs. 3A-11 below.

Capsule body 12 may comprise any suitable material, such as metal or plastic. Arms 10 may also comprise materials such as metal or plastic. In some embodiments, the arms comprise piezoelectric materials, so as to enable the arms to move and vibrate, as described below.

In some embodiments, insertion of the capsule is visualized using an imaging system, such as an ultrasound imaging system, an x-ray imaging system or any other suitable imaging modality. The imaging system enables the physician to track the location of the capsule in the body. For this purpose, the capsule may comprise material that is clearly visible to the imaging modality used.

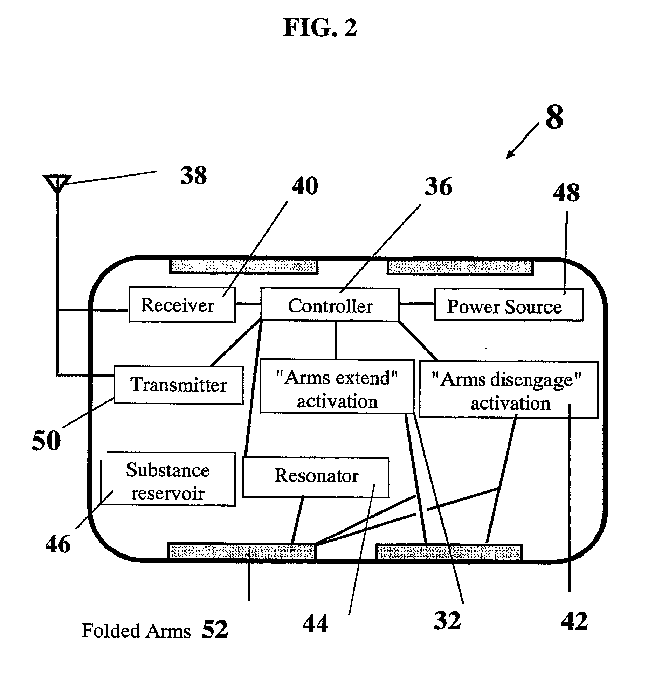

Capsule 8 is controlled by an external console 14. Console 14 comprises an input device, such as a keypad 18, using which the physician enters commands for controlling the capsule. A transmitter inside console 14 translates the commands into control signals and transmits the control signals via a transmit antenna 15 to a miniature receiver (shown in Fig. 2 below) fitted in the capsule. The transmitter typically transmits a low power signal, and the antenna is typically positioned in close proximity to the patient body, in order to eliminate effects on neighboring patients who may have a similar capsule operating in their body and to minimize the need for different frequencies to be used by different transmitters. In an alternative embodiment, different transmitter-capsule pairs may use different frequencies.

In some embodiments, the physician can activate the capsule, i.e., instruct the capsule to expand, by transmitting an appropriate command from console 14. The physician may track the capsule using the imaging system as it travels through the gastro-intestinal tract. When the capsule reaches the location of interest, in this case the patient's stomach, the physician instructs the capsule to expand. The capsule receives the command and releases arms 10 to their expanded position. As a result, the capsule anchors at the desired location.

Once anchored and activated, the capsule can be used to create local and systemic effects through several mechanisms. Since the distal ends of arms 10 are in contact with the gastrointestinal mucosa (in this case -the inner lining surface of the stomach) they apply pressure, i.e., tactile, mechanical and/or nociceptive stimulation, at the points of contact. These points are often dispersed over a large surface area. The various modes of stimulation applied by the arms to the tissue may cause secretion of hormones having both local and peripheral effects, ultimately leading to reduced caloric intake. Similarly, stimulation of local receptors and nerve endings within the tissue in proximity to the arms will create a neuronal discharge to the central nervous system, thus causing systemic effects in multiple physiological systems.

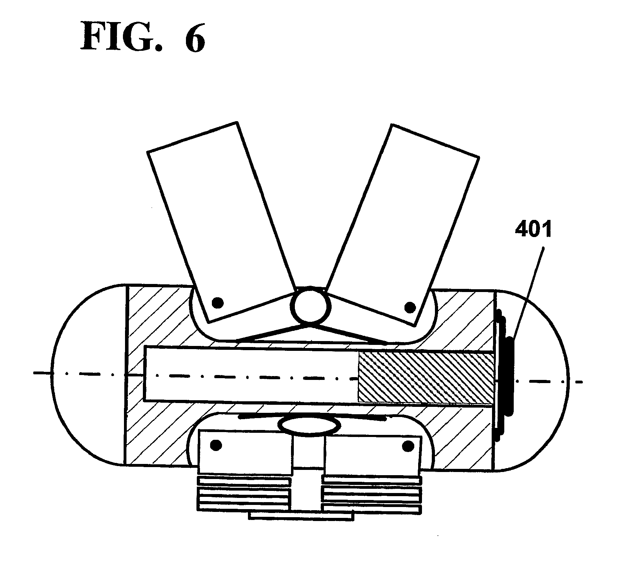

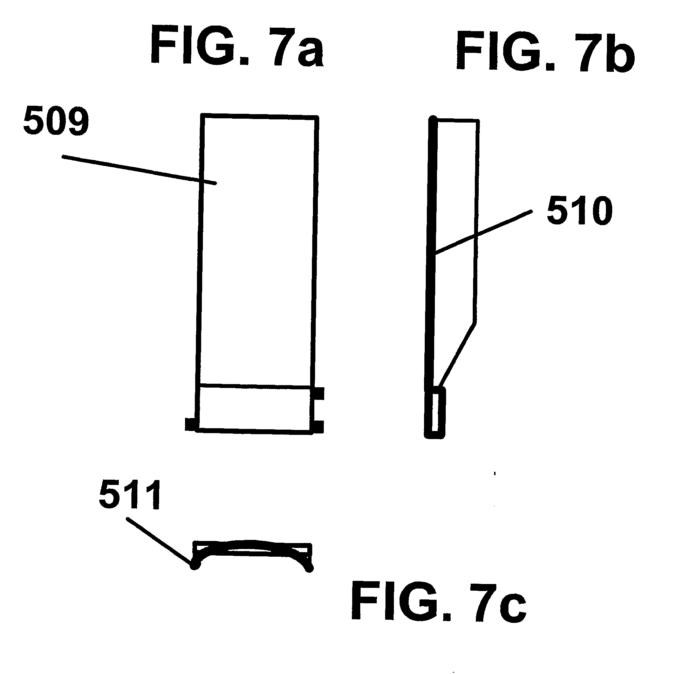

In some embodiments, the stimulation applied by arms 10 is enhanced by vibrating the arms. For example, the expanded capsule structure may be vibrated at a frequency close to the resonant frequency of the arms. Typically, the structure is expected to have a resonant frequency in the range of 0.1 to 10Hz. Vibrating the arms can be carried out using a resonator within the capsule, for example, or using arms made of piezoelectric materials. An exemplary capsule configuration comprising a resonator is described in Fig. 6 below. Exemplary arms made of piezoelectric materials are shown in Fig. 7 below. It should be noted that the tissue stimulation effects described above are significantly stronger than the effect of the physical size of the capsule *per* se in causing the desired physiological effects.

The stimulation applied to the tissue by the arms may cause several effects. In some cases, a nociceptive stimulation of tissue creates a discharge of hormones, peptides and/or cytokines having endocrine, autocrine and/or paracrine effects. Additionally or alternatively, applying light pressure on tissue of interest creates neurally-mediated physiological systemic and/or local effects via afferent and efferent fibers. Applying pressure by the arms may also affect mechanically-activated tissue receptors, producing systemic and/or local effects. Further additionally or alternatively, applying local tissue contact may alter the physical tissue properties such as permeability, temperature and surface texture. Furthermore, a localized contact with tissue typically alters its volume and changes its intra-luminal pressure, thus inducing a counter-dilation reaction.

In some cases, the tissue stimulation produced by capsule 12 may reduce the secretion of orexogenic hormones, such as gherlin and other peptides or molecules, by imitating a constant presence of an undigested element in the stomach. The nociceptive stimulation may also induce the secretion of hormones from the distal gastrointestinal tract, such as PYY 3-36, GLP-1, enterostatin or other peptides or molecules, which inhibit food intake.

In some embodiments, the capsule can locally discharge various substances that affect the central nervous system and aid in suppressing the patient's appetite. These substances may induce local and distal effects of hormonal and neural discharge. Such substances may comprise any suitable chemical substance, such as a molecule, peptide, protein or other pharmacologic substance having an appetite suppression effect. For example, the administered substance may comprise a fatty acid derivative such as linoleic acid. Some peptides that can be discharged by the capsule may induce the secretion of gut hormones, such as cholecystokinin (CCK), from the upper gastrointestinal tract, thus reducing caloric intake.

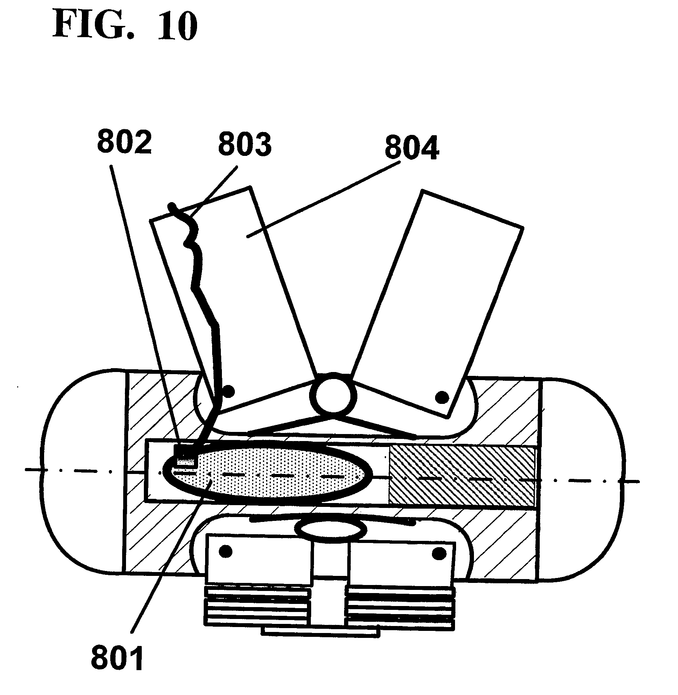

Since the capsule delivers the substance locally, extremely small quantities of the substance are sufficient for inducing the desired effect. In some embodiments, the capsule comprises a miniature container (shown in Fig. 10 below) containing the substance in question. Alternatively, the outer surface of the arms and/or the capsule body may be impregnated with the substance. In these embodiments, the substance is released using the endogenous motion of the arms and the capsule or by a sustained release mechanism.

In some embodiments, the tissue stimulation and/or substance discharge can be controlled and customized. Mechanical tissue stimulation and/or substance administration can be activated and deactivated on demand by transmitting commands from the external console to match a desired treatment profile. Thus, the physician is able to alter the treatment profile over time and to customize the profile to the needs of a particular patient. Altering the treatment profile also helps to reduce adaptation and resistance to the treatment by the patient's body. Alternatively, a particular treatment profile can be pre-programmed in the capsule.

In order to remove capsule 12 from the patient's body, the capsule comprises a disassembly mechanism, which causes arms 10 to disengage from capsule body 12. The disassembly mechanism can be activated, for example, in response to a command transmitted from external console 14. Additionally or alternatively, the disassembly mechanism can be activated after a pre-programmed time interval, when the capsule power source is close to exhaustion or reaches a certain capacity threshold, when the substance administered by the capsule runs out or when any other suitable condition is fulfilled.

Once disassembled, the different capsule elements pass through the digestive tract naturally and are excreted through the patient's digestive system. Typically, the different parts of the capsule comprise materials that can be clearly imaged by the imaging modality used (e.g., ultrasound), so that the physician is able to verify that no parts remain in the body.

Additionally or alternatively, some aspects of the treatment profile can sometimes be controlled during everyday use, such as by the patient himself. For example, in some embodiments the patient is provided with a personal control unit 16, which allows control over the operation of capsule 8. Unit 16 comprises an input device, such as a keypad 17, for this purpose. Typically, the personal control unit comprises a subset of the functions and elements of console 14. For example, the patient can use the personal control unit to activate and deactivate the vibration of arms 10, in order to enable normal caloric intake at certain times and reduce caloric intake at other times. Providing access to other functions of the personal control unit is customizable and user-dependent, enabling different users to have access to different sets of functions and activities of the device. Activating the capsule disassembly may or may not be enabled in unit 16.

Fig. 2 is a block diagram that schematically illustrates functional elements of capsule 8, in accordance with an embodiment of the present invention. Some elements in Fig. 2 are optional, i.e., different configurations of capsule 8 may comprise only a subset of the elements. Several exemplary capsule configurations are described in detail further below.

Capsule 8 comprises a controller 36, which manages the operation of the capsule and activates its elements. Controller 36 may comprise a microprocessor running suitable software code. Alternatively, the controller may be implemented using hardware or firmware, such as using an application specific integrated circuit (ASIC) or field programmable gate array (FPGA). Further alternatively, the controller may be implemented using a combination of hardware and software functions. Typically, the controller comprises one or more pulse generators or other drivers that activate the different capsule elements, including the piezoelectric functions.

Capsule 8 comprises an antenna 38 and a receiver 40, which receive the control signals transmitted from console 14. (In the description that follows, external commands are described as being transmitted by console 14. As noted above, these commands may similarly be transmitted by personal control unit 16.) The receiver decodes the transmitted commands and provides them to controller 36. The capsule further comprises an arms extend activation module 32 and an arms disengage activation module 42.

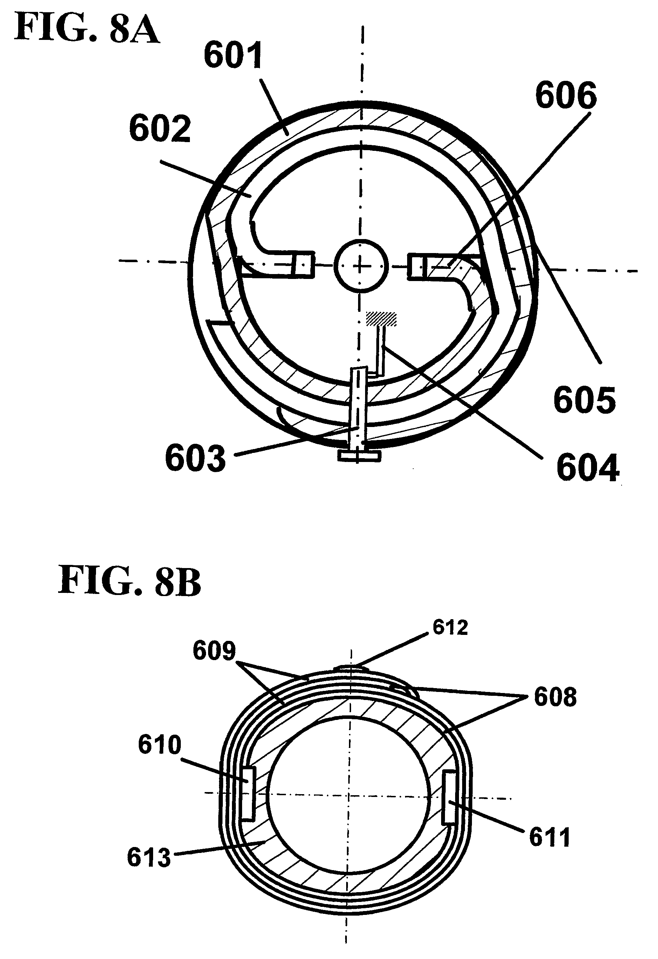

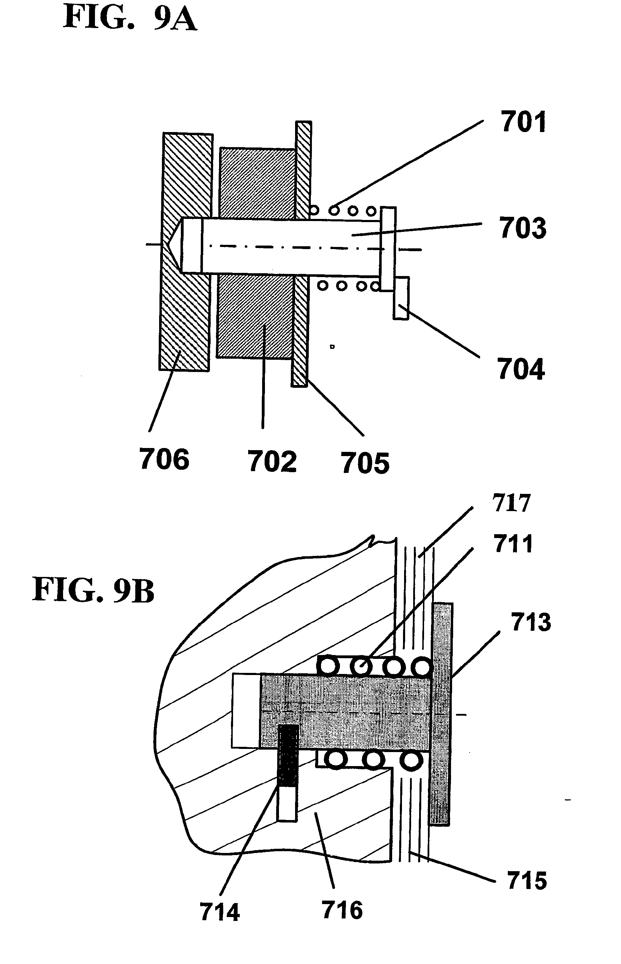

When instructed by controller 36, module 32 releases self-extending arms 10 so as to enable them to open to their expanded position. When instructed by the controller, module 42 disassembles the arms from their base, thus disengaging the arms from the capsule body, to allow the capsule to leave the body. In general, releasing and/or disassembling the arms can be triggered by different conditions, such as after a predetermined time or based on an external command. Exemplary release and disassembly mechanisms are shown in detail in Figs. 8A, 8B, 9A and 9B below.

In some embodiments, capsule 8 comprises a resonator 44, which vibrates the expanded capsule structure, as described above. Resonator 44 may comprise a commercial disk-shaped resonator, a bending resonator, a twist resonator, or any other suitable resonator known in the art. The resonator is activated and deactivated by controller 36. Alternatively, e.g., when capsule 8 comprises piezoelectric arms, the arm vibrations are activated, generated and deactivated by controller 36, as preprogrammed, or alternatively triggered by the console 14 or unit 16.

In some embodiments, the capsule comprises a miniature substance reservoir 46, which holds a substance that is locally administered by the capsule. The discharge of substance from reservoir 46 is activated and deactivated by controller 36. An exemplary configuration comprising a substance reservoir is shown in Fig. 10 below.

In some embodiments, capsule 8 comprises a transmitter 50, which transmits data provided by controller 36 to an external receiver (not shown). The data transmitted by transmitter 50 may comprise, for example, acknowledgements of the external commands received and performed by the capsule. Additionally or alternatively, the transmitter may transmit telemetry and status data such as battery status and failure indications, and/or any other suitable information of interest. In the configuration of Fig. 2 the transmitter uses antenna 38 for transmitting the data, although in alternative configurations separate transmit and receive antennas can also be used.

Capsule 8 comprises a power source 48, which supplies electrical power to the different capsule elements. Power source 48 may comprise one or more redundant batteries for enhancing capsule reliability. In some embodiments, controller 36 is able to monitor the voltage and/or current of the power source, so as to sense when the power source is weak or exhausted.

In an alternative embodiment, some or all of the energy for powering capsule 8 can be provided by transmitting radio frequency (RF) energy to the capsule. RF energy is typically transmitted using an external coil, which may be worn by the patient or be part of console 14 or unit 16. The RF transmission may be used to power the capsule directly and/or to charge a battery in the capsule. In these embodiments, power source 48 comprises a suitable coil for receiving the transmitted RF energy, as well as suitable rectification circuitry, as is known in the art.

### CAPSULE MECHANICAL CONFIGURATIONS

Figs. 3A-11 below show several exemplary mechanical configurations of self-extracting capsules and some of their internal mechanisms.

Fig. 3A is a longitudinal cross-section of an intrabody capsule, in accordance with an embodiment of the present invention. The capsule comprises a capsule body 101, comprising metal, plastic or any other suitable material. Two arms 102 and 109 are shown fully-extended. Two arms 102A and 109A are tightly wrapped around the capsule body. The arms may comprise any suitable elastic material, such as various metals and plastics. In some embodiments described below, the arms comprise a piezoelectric material. The arms are constructed so that their natural, unrestrained state is extended. In other words, when no force is applied to the arms, the arms will remain in the extended position.

Arms 102A and 109A are shown wrapped several times around the capsule body. Each arm is fastened to capsule body 101 so as to rotate around a respective pivot 103. The wrapped arms are pressed against a compressed spring 104A and held by a lock/release mechanism 110. The lock/release mechanism comprises a pin, a latch and a cover 107. One or more such mechanisms lock the folded arms to the capsule body. Cover 107 may comprise one or more thin parts made of metal, polymer and/or fabric. Exemplary lock/release mechanisms are shown in greater detail in Figs. 8A, 8B, 9A and 9B below.

The latch of mechanism 110 is actuated to release the arms by a controller 108, similar to controller 36 of Fig. 2 above. The latch can be actuated using a shape memory alloy (SMA) element, a motor, a piezoelectric element, a solenoid, or using any other suitable actuation device known in the art. When the latch is actuated, the pin of mechanism 110 is pushed out of the capsule, releasing cover 107 and allowing the arms to extend. Spring 104A further pushes the arms to rotate around pivot 103 until the arms reach the fully-extended and sidewise-tilted positions. A spring 104 is shown in its uncompressed form, with arms 102 and 109 fully extended. Spring 104 is shown as a central helical torsion spring, although a leaf spring, a torsion spring on pin 103 or any other suitable spring known in the art can also be used.

When extended, arms 102 and 102A are located in a certain position, and arms 109 and 109A are located in a different position. As can be appreciated, the distal ends of arms 102 and 109 are distant from one another, thus providing both anchoring and tissue stimulation over a set of widely-dispersed points on the inner stomach surface. A receiver and antenna 106 and a battery 105 are also shown. The distal ends of arms 102 and 109 are curved (not shown), so as to avoid tissue damage and blocking of orifices.

In some embodiments, pivot 103 is also used as part of the capsule disassembly mechanism. When instructed by the controller, the disassembly mechanism removes pivot 103 from its place. As a result, the arm is disconnected from the pivot and disengages from the capsule body. Exemplary disassembly mechanisms are shown in Figs. 9A and 9B below.

Fig. 3B is a longitudinal cross-section of an intrabody capsule, in accordance with another embodiment of the present invention. In the present example, arms 111 and 112 comprise flexible arms that are wrapped over conically-shaped parts 118 of the capsule body. Thus, when the arms are released they unfold into curved shapes having distal ends that are distant from one another, without requiring the use of springs as in the embodiment of Fig. 3A above. Arms 111 and 112 may comprise piezoelectric material, so that they can be electrically controlled and vibrated without the use of springs or other resonators in the capsule. The distal ends of arms 111 and 112 are curved and are designed to be atraumatic, so as not to damage the tissue in their vicinity or block natural orifices.

A disassembly ball-lock mechanism 116 comprises springs 117. When actuated, the balls release domes 115 and 119. Springs 117 then push the two domes outwards along the central axis of the capsule. The movement of the domes frees arms 111 and 112 to disengage from the capsule body. As noted above, mechanism 116 can be actuated by a SMA element, motor, piezoelectric element, solenoid or any other suitable actuation device. In the present example, mechanism 116 comprises a ball-lock mechanism. Alternatively, mechanism 116 may comprise any other suitable locking mechanism known in the art.

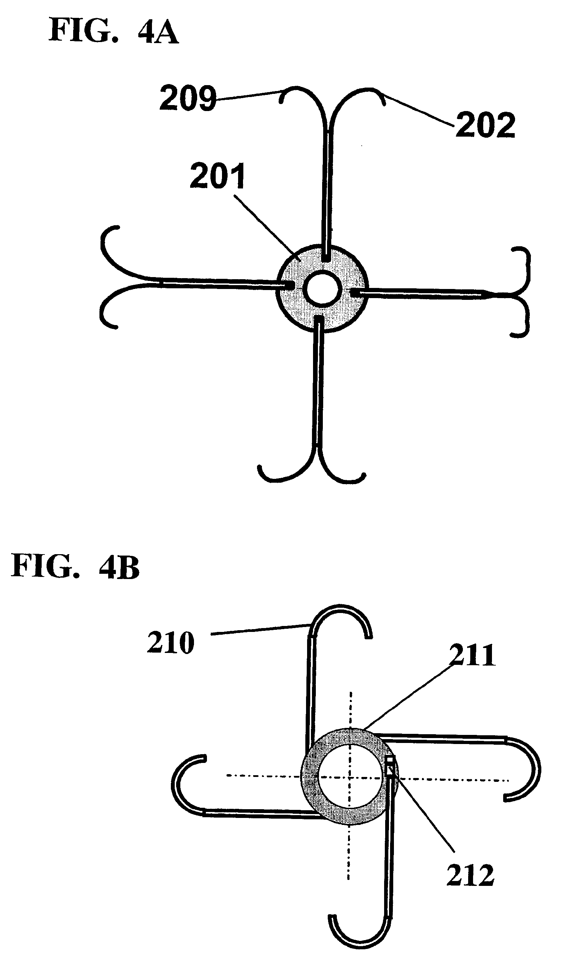

Fig. 4A is a transverse cross-section of an intrabody capsule, in accordance with an embodiment of the present invention. The figure shows the capsule with its arms in the fully extended position. In the present example, eight arms are attached to a capsule body 201. Two arms 202 and 209 are shown curved in different directions, although each of the eight arms may alternatively be curved in any desired direction or plane. The curvature of the distal ends of the arms helps to prevent tissue damage or blockage of orifices. When folded, the arms are wrapped around the perimeter of capsule body 201 and held in place by a suitable lock/release mechanism (not shown).

Fig. 4B is a transverse cross-section of an intrabody capsule, in accordance with another embodiment of the present invention. Unlike the configuration of Fig. 4A above in which the arms bases are perpendicular to the surface of the capsule body, in the present example arms 210 are tangent to a capsule body 211. The tangent arm configuration reduces the strain at the point of connection between the arms and the capsule body when the arms are in the folded position. The tangent arm configuration may also simplify and smoothen the disengagement of the arms when the capsule is disassembled.

The disassembly mechanism of the capsule of Fig. 4B comprises two domes or caps (not shown), similar to domes 115 and 119 of Fig. 3B above. Each arm 210 comprises a base 212. When the domes are pushed aside during disassembly of the capsule, bases 212 are free to disengage from capsule body 211 so as to separate the arms from the body.

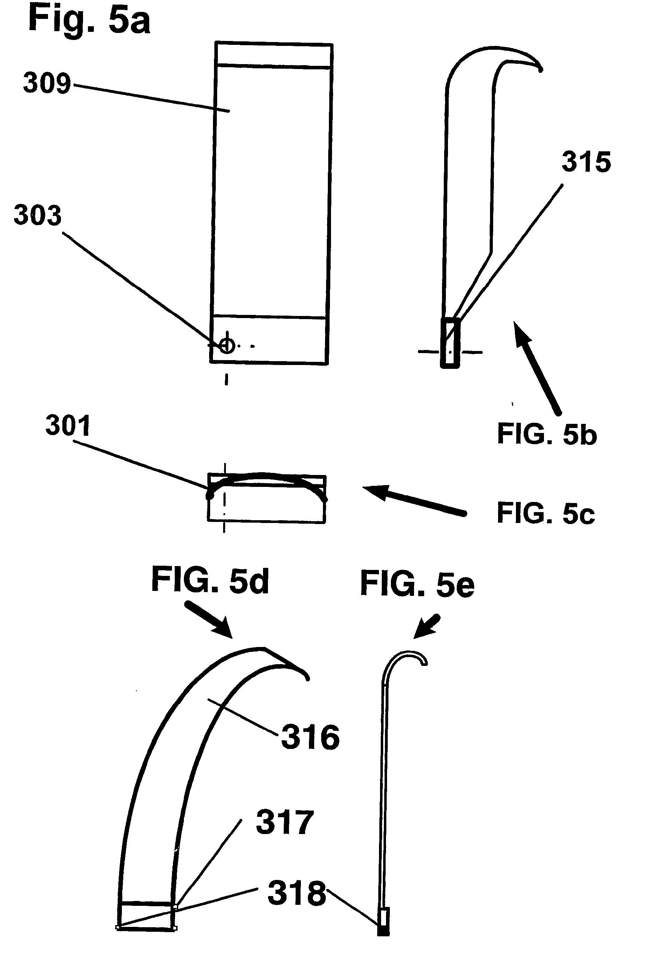

Fig. 5A is a schematic frontal view of a self-extending arm 309, in accordance with an embodiment of the present invention. Arms of this kind can be used, for example, in the capsule configurations of Figs. 3A, 4A and 4B above. Arm 309 comprises a flexible, elastic material such as metal, plastic or piezoelectric material.

Figs. 5B and 5C respectively show side and top views of arm 309. As can be seen in Fig. 5C, the arm has a slightly curved profile 301 along its entire length, so as to provide some structural rigidity to the arm when extended. The curved profile of the arm is flattened when the arm is wrapped around the capsule body. Alternatively, other stiffening geometries can also be used. As shown in Fig. 5B, the arm comprises a thickened bottom section 315, which provides a firm attachment to the capsule body. The bottom section comprises a pivot 303, similar to pivot 103 of Fig. 3A above.

Figs. 5D and 5E are schematic illustrations of a self-extending arm 316, in accordance with an alternative embodiment of the present invention. Fig. 5D is a frontal view, and Fig. 5E is a side view of arm 316. In the present example, the arm has curved sides, so as to enable the arm to be wrapped around a conical part of the capsule body, and then spread outward longitudinally when released, as shown in Fig. 3B above. Arm 316 is stiffened using a curved profile along its entire length, similarly the profile shown in Fig. 5C above. Arm 316 typically comprises a suitable piezoelectric material, as described further herein below, but may alternatively be made of metal or plastic material. When made of piezoelectric materials, the arms may comprise a feedback element, which enables the controller to regulate the vibration in the resonance frequency of each arm separately. The feedback mechanism is described in greater detail further below.

The bottom section of arm 316 comprises one or more cantilever extensions. In Fig. 5D, two extensions denoted 317 and 318 are shown. The extensions are fitted into respective cavities (not shown) in the capsule body and domes, and anchor the arm to the capsule body. Typically, the extensions facing the perimeter of the capsule body have rounded ends. When the capsule is to be disassembled, the domes are pushed aside and the extensions are free to eject out of the cavities, so as to disengage the arms.

Fig. 6 is a longitudinal cross section of an intrabody capsule, in accordance with yet another embodiment of the present invention. A resonator 401, in the present example comprising a piezoelectric disk, is fitted in the capsule body. Alternatively, any other suitable resonator can be used. As explained above, the resonator vibrates the extended arms, typically at a frequency near the resonant frequency of the arms.

In many cases, each arm has a slightly different resonant frequency. Thus, in some embodiments, the resonator is actuated to produce random vibrations within a relatively narrow frequency band that is expected to include the different resonant frequencies of the arms. Although resonator 401 is shown fitted in a capsule similar to the configuration of Fig. 3A above, similar resonators can be fitted in any other suitable capsule configuration.

Figs. 7A-7C are schematic illustrations of a self-extending and vibrating arm 509, in accordance with an embodiment of the present invention. Fig. 7A is a frontal view, Fig. 7B is a side view and Fig. 7C is a top view of arm 509. The geometry of arm 509 is similar to the geometry of arm 309 of Figs. 5A-5C above, although any other suitable arm geometry can be used. Arm 509 comprises (i.e., is at least partially made of) a piezoelectric material.

In order to generate vibration, controller 36 applies an alternating current (AC) voltage to the piezoelectric material of the arm. As a result of the piezoelectric effect, the arm vibrates in accordance with the frequency of the AC voltage.

The material may comprise a piezoelectric polymer such as Polyvinyldifluoride (PVDF), a composite material such as macro-fiber composite (MFC), or any other suitable material having piezoelectric properties. The arm may be constructed as a uni-morph, a bimorph or any other suitable piezoelement known in the art. The piezoelectric effect may cause the arm to twist, bend, extend or otherwise modify its shape.

As noted above, the resonant frequency may vary from one arm to another. In some embodiments, each arm may comprise a feedback piezoelectric element. Using the feedback provided by these elements, controller 36 drives each arm at a different frequency that matches its individual resonant frequency. For example, controller 36 may comprise a feedback mechanism, which senses the vibration frequency of the feedback element of each arm and adjusts the frequency of the AC voltage driving this arm, attempting to maximize the vibration magnitude.

Fig. 8A is a transverse cross-section of an intrabody capsule, in accordance with an embodiment of the present invention. The transverse cross-section of Fig. 8A corresponds to the configuration of Fig.4A above. The figure shows two flexible arms 601 (shaded) and 602 wrapped around the capsule body within a body contour 605. The arms are connected to the capsule body at arm bases 606. A lock/release mechanism comprises a pin 603 that is pushed out by a spring (not shown) and a shape memory alloy (SMA) beam 604. When beam 604 is actuated by the controller, pin 603 is released and separates from the capsule. As a result, arms 601 and 602 are free to self-extend. Alternatively, any other suitable actuation device can be used instead of SMA beam 604, such as, for example, a solenoid, a motor or a piezoelectric device. In alternative embodiments, a larger number of arms and/or longer arms that are wrapped several times over the perimeter of the capsule body can be used.

Fig. 8B is a transverse cross-section of an intrabody capsule, in accordance with another embodiment of the present invention. The transverse cross-section of Fig. 8B corresponds to the configuration of figure 4B above. The figure shows two flexible arms 608 and 609 wrapped around the capsule body and held in place by the upper part of a locking element 612. The locking element is shown in greater detail in Fig. 9B below. Although Fig. 8B shows two arms, any number of arms can be used.

Arm 608 comprises a thickened base 610, which is held in place after the extension of the arms. When released, the base disengages the entire arm from the capsule body. Similarly, arm 609 comprises a thickened base 611. The arms need not necessarily be of the same length. In the exemplary embodiment of Fig. 8B, a single locking element 612 is used to hold all arms. Alternatively, two or more locking elements may be used.

Fig. 9A is a cross-section of a lock and release mechanism of a self-extracting arm, in accordance with an embodiment of the present invention. The figure shows a pivot 703, around which an arm 702 rotates with respect to capsule body parts 705 and 706. Only parts of arm 702 and body 705 that are in close proximity to the pivot are shown. This mechanism can be used, for example, as pivot 103 in the capsule of Fig. 3A above.

Pivot 703 compresses a spring 701 and is held by a latch 704. When latch 704 is actuated (i.e., moved aside) by the controller, spring 701 pushes pivot 703 out of the capsule body. As a result, arm 702 is no longer held by pivot 703 and is now separated from capsule body 705. The latch can be actuated using any of the actuation methods described above.

Fig. 9B is a cross-section of a lock and release mechanism of a self-extending arm, in accordance with another embodiment of the present invention. The upper part of a central pin 713 holds folded arms 715 and 717 in the folded position against a capsule body 716. Pin 713 is locked in place by a detent 714. When the detent releases the pin after being triggered by the controller, a spring 711 pushes the pin outwards and releases the arms. The detent may comprise shape memory alloy (SMA), which bends or changes its shape by electric heating, a solenoid, a piezo-element or be moved by a motor.

Fig. 10 is a longitudinal cross section of an intrabody capsule, in accordance with an embodiment of the present invention. In the present example, the capsule comprises a reservoir 801, which is filled with a substance to be locally administered by the capsule. An electric valve 802, which is controlled by the controller, starts and stops the flow of substance out of the reservoir. One or more thin, flexible tubes 803 transfer the substance to the distal ends of arms 804, so as to discharge the substance adjacently to the tissue. The tubes are typically embedded in the arms. The valve may be opened periodically by the controller in accordance with a pre-programmed profile. Alternatively, the valve can be opened on demand, by transmitting a suitable command from the external console.

In some embodiments, the reservoir is pre-pressurized so that the substance flows freely when valve 802 is opened. Alternatively, the substance can be discharged using natural diffusion. As noted above, substances can alternatively be discharged locally by impregnating the outer surface of the capsule body and/or arms with substance. As an example, the arms can be impregnated by Hexacapron and the substance released in the vicinity of bleeding tissue. As another example, the arms may be impregnated by Urokinase in order to treat blood clot. Additionally or alternatively, the arms can be impregnated by any other suitable medication to influence the reactions in the target organ.

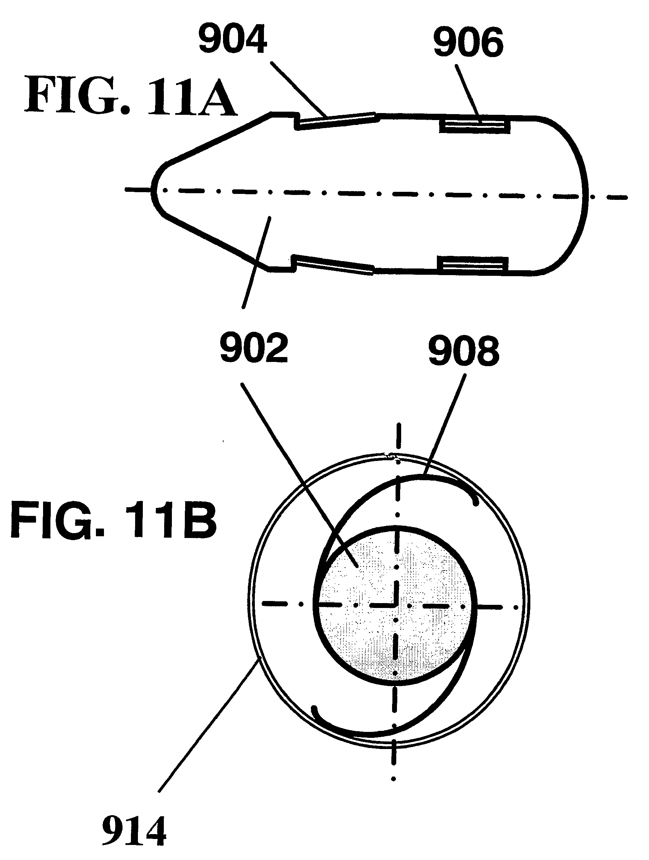

Figs. 11A and 11B respectively show a longitudinal cross section and a transverse cross section of an intrabody capsule 902, in accordance with another embodiment of the present invention. Several intrabody capsule products are known in the art. Some known capsules are fitted with video cameras or other sensors and devices. In some embodiments, a known capsule can be modified to include self-unfolding arms, in accordance with the embodiments described herein. A desirable mode of operation for camera capsules is the possibility to anchor the capsule in a certain location in the gastrointestinal tract, and to move it slightly forwards and backwards around a location of interest.

The location of interest may be selected by a physician based on transmitted images or automatically by the capsule using suitable pattern recognition software. Based on the indications of the pattern recognition software, the capsule may also transmit alerts to console 14.

In Fig. 11A, capsule 902 comprises arms 904 and 906, which are shown in their tightly-packed position. Unlike some of the embodiments described above, in the present example the arms are held, expanded and contracted using a piezoelectric effect. As such, capsule 902 does not comprise a locking mechanism. In Fig. 11A arms 906 are shown parallel to the capsule body, while arms 904 are tilted at a certain angle with respect to the capsule body. In alternative embodiments, arms 906 can be tilted in the opposite direction or at any desired angle.

In Fig. 11B the arms (denoted 908) are shown in their expanded position. The capsule controller applies a suitable voltage to the piezoelectric material of the arms. As a result, the arms expand until they push against an outer tissue 914. The controller can cause the arms to contract by removing the voltage or by applying a different voltage to the piezoelectric arms. The controller may control each arm separately, or it may alternatively control groups of arms or all arms simultaneously.

Depending on the application, the tissue may comprise the colon, the small intestine or any hollow organ or tubular viscus within the body. The configuration of Figs. 11A and 11B is particularly suitable for scenarios in which a modest deflection of the arms is sufficient to ensure anchoring, as shown in Fig. 11B. Such an application may comprise, for example, anchoring the capsule in the small intestine, whose diameter is only slightly larger than the diameter of the capsule or in a narrowed or blocked blood vessel. However, capsules similar to capsule 902 may also be used in larger diameter organs such as the colon.

Although the embodiments described herein mainly address a capsule, which is anchored in the gastrointestinal tract and produces tissue stimulation for reducing caloric intake, the principles of the present invention can also be used for additional applications. For example, similar capsules can be inserted into other luminal organs or systems, such as into a blood vessel or into the urinary tract. The dimensions of the capsule and arms should be adjusted to fit the organ of interest. As another example, a capsule can be inserted surgically through a narrow orifice and positioned within or adjacent to the tissue of interest. Devices can be inserted, either invasively or non-invasively, into any suitable target organ, such as but not limited to the central nervous system, intra-abdominal organs, thoracic organs and limbs. In such cases, the device may be capsule-shaped or have any other shape suitable for use in the specific target organ.

In addition to reducing caloric intake, applying mechanical localized tissue stimulation can be used to treat other gastrointestinal conditions such as localized bleeding or inflammation. Localized substance delivery can also be used to reach specifically high concentrations of the substance while preventing systemic effects.

In some embodiments, certain medical conditions can be identified by examining the arms after they are disposed of by the patient body. For example, tissue residues deposited on the arms can be examined. The arms can be examined for the occurrence of certain chemical reactions, such as contact with blood and/or a change in PH value.

Additionally or alternatively, certain medical conditions, such as internal bleeding, can be sensed by the arms when the capsule is anchored in the target organ. The capsule can transmit to the external receiver indications of such conditions.

In some embodiments, controlling the motion of the extended arms can also be used to navigate the capsule inside the body, such as along the gastrointestinal tract. Navigation can either be autonomic or controlled by the external console. By regulating the extension of each arm separately, a movement along the capsule axis can be achieved.

It will thus be appreciated that the embodiments described above are cited by way of example, and that the present invention is not limited to what has been particularly shown and described hereinabove.

## Claims

1. A device for insertion into an organ of a patient, comprising:
a capsule body (12);
one or more arms (10), which are attached to the capsule body and are arranged to expand from the capsule body in the organ so as to contact an inner surface of the organ and anchor the device at the desired location;
a resonator (44) attached to the capsule body, which is arranged to vibrate the one or more arms;
a disassembly mechanism (42), which is arranged to disengage the one or more arms from the capsule body so as to allow the capsule body and the one or more arms to be disposed of; and
a controller (36), which is arranged to activate and deactivate said resonator, wherein
when extended, the one or more arms are arranged to apply at least one of a mechanical, a tactile and a nociceptive stimulation at respective contact points in the organ and the one or more arms are arranged to transfer a mechanical vibration to the respective contact points.

2. The device according to claim 1, wherein the capsule body is arranged to be swallowed by the patient, and wherein the one or more arms are arranged to expand from the capsule body in the stomach.

3. The device according to claim 1 or claim 2, wherein the one or more arms comprise at least one of a metal, a plastic, and a piezoelectric material.

4. The device according to any of claims 1 to 3 and wherein the resonator is arranged to vibrate the one or more arms with a random vibration having frequencies within a narrow bandwidth containing respective resonant frequencies of the one or more arms.

5. The device according to any of claims 1 to 4, wherein said controller activates said disassembly mechanism responsively to a disassembly condition, wherein the disassembly condition comprises at least one of a predetermined time schedule, a low power condition of a power source of the device, and an external command transmitted to the device.

6. The device according to claim 5, wherein the disassembly mechanism comprises at least one of a shape memory alloy (SMA) element, a motor, a piezoelectric element, a solenoid, a ball lock mechanism, and a detent.

7. The device according to any of claims 1 to 6, and also comprising a power source (48) comprising at least one of a battery, a rechargeable battery, and a radio frequency (RF) coil arranged to accept externally-transmitted RF energy.

8. The device according to any of claims 1 to 7, wherein the device is arranged to discharge a substance at the desired location within the organ.

9. The device according to claim 8, and also comprising a container holding the substance fitted in the capsule body.

10. The device according to claim 9, wherein the substance is impregnated in at least one of an outer surface of the capsule body and the one or more arms.

11. The device according to any of claims 1 to 10, and also comprising:
a transmitter (50); and
an antenna (38) and a receiver (40), which are arranged to receive commands transmitted to the device from an external transmitter in an external console (14); and
wherein said controller (36) is arranged to control the operation of the device responsively to the commands and is also arranged to transmit via the transmitter (50) data provided by the controller to an external receiver in the console (14).

12. The device according to claim 1, wherein the capsule body and the one or more arms are arranged to be inserted into at least one of a blood vessel, a part of a genitourinary tract, a part of a central nervous system, an intra-abdominal organ, a thoracic organ and a limb of the patient.

13. The device according to claim 1, wherein the device is arranged to be inserted into the organ using an endoscope or invasive means.

## Patentansprüche

1. Vorrichtung zum Einführen in ein Organ eines Patienten, umfassend:
- einen Kapselkörper (12);
- einen Arm oder mehrere Arme (10), die an dem Kapselkörper befestigt sind und ausgestaltet sind, um von dem Kapselkörper aus im Organ zu expandieren, um so eine Innenfläche des Organs zu kontaktieren und die Vorrichtung an der gewünschten Stelle zu verankern;
- einen an dem Kapselkörper befestigten Resonator (44), der ausgestaltet ist, um den Arm oder die Arme zum Vibrieren zu bringen;
- einen Demontagemechanismus (42), der ausgestaltet ist, um den Arm oder mehrere Arme von dem Kapselkörper zu lösen, damit so der Kapselkörper und der Arm oder mehrere Arme entsorgt werden können; und
- eine Steuerung (36), die ausgestaltet ist, um den Resonator zu aktivieren und zu deaktivieren, wobei
- der Arm oder mehrere Arme in ausgestrecktem Zustand ausgestaltet sind, um zumindest entweder eine mechanische, eine taktile oder eine nozizeptive Stimulation an entsprechenden Kontaktstellen in dem Organ anzulegen und der Arm oder mehrere Arme ausgestaltet sind, und um eine mechanische Vibration an die entsprechenden Kontaktstellen zu übertragen.

2. Vorrichtung nach Anspruch 1, bei dem der Kapselkörper ausgestaltet ist, damit ihn der Patient hinunterschlucken kann und bei dem der Arm oder mehrere Arme ausgestaltet sind, um von dem Kapselkörper im Magen zu expandieren.

3. Vorrichtung nach Anspruch 1 oder Anspruch 2, bei der der Arm oder mehrere Arme zumindest entweder ein metallisches, ein Kunststoff- oder ein piezoelektrisches Material umfassen.

4. Vorrichtung nach einem der Ansprüche 1 bis 3 und bei der der Resonator ausgestaltet ist, um den Arm oder mehrere Arme mit einer nach dem Zufallsprinzip ausgewählten Vibration zum Vibrieren zu bringen, wobei die Vibration Frequenzen innerhalb einer engen Bandbreite aufweist, die entsprechende Resonanzfrequenzen des Arms oder mehrerer Arme enthält.

5. Vorrichtung nach einem der Ansprüche 1 bis 4, bei der die Steuerung den Demontagemechanismus in Reaktion auf einen Demontagezustand aktiviert, bei dem der Demontagezustand zumindest entweder einen vorgegebenen Zeitablaufplan, einen niedrigen Spannungszustand einer Stromquelle der Vorrichtung oder einen an die Vorrichtung übertragenen externen Befehl umfasst.

6. Vorrichtung nach Anspruch 5, bei der der Demontagemechanismus zumindest entweder ein Element einer Formgedächtnislegierung (SMA), einen Motor, ein piezoelektrisches Element, einen Solenoiden, einen Kugelverschlussmechanismus oder eine Arretierung umfasst.

7. Vorrichtung nach einem der Ansprüche 1 bis 6 und ebenfalls umfassend: eine Stromquelle (48), umfassend zumindest entweder eine Batterie, eine wiederaufladbare Batterie oder eine Hochfrequenz-Spule, um von außen übertragene Hochfrequenz-Energie entgegenzunehmen.

8. Vorrichtung nach einem der Ansprüche 1 bis 7, bei dem die Vorrichtung ausgestaltet ist, um eine Substanz an einer gewünschten Stelle innerhalb des Organs abzugeben.

9. Vorrichtung nach Anspruch 8 und ebenfalls umfassend: ein Behältnis, das die Substanz enthält, eingebaut im Kapselkörper.

10. Vorrichtung nach Anspruch 9, bei der zumindest entweder die Außenfläche des Kapselkörpers oder der Arm bzw. mehrere Arme mit der Substanz durchtränkt sind.

11. Vorrichtung nach einem der Ansprüche 1 bis 10 und ebenfalls umfassend:
- einen Sender (50) und
- eine Antenne (38) und einen Empfänger (40), die ausgestaltet sind, um von einem externen Sender an die Vorrichtung übertragene Befehle in einem externen Bediengerät (14) zu empfangen; und
wobei die Steuerung (36) ausgestaltet ist, um den Betrieb der Vorrichtung in Reaktion auf die Befehle zu steuern, und ebenfalls ausgestaltet ist, um Daten über den Sender (50) zu übertragen, die von der Steuerung an einen externen Empfänger in dem Bediengerät (14) bereitgestellt wurden.

12. Vorrichtung nach Anspruch 1, bei dem der Kapselkörper und der Arm oder mehrere Arme ausgestaltet sind, um entweder zumindest in ein Blutgefäß, einen Teil eines Urogenitaltraktes, einen Teil eines Zentralnervensystems, ein intra-abdominelles Organ, ein Thoraxorgan oder ein Gliedmaß eines Patienten eingeführt zu werden.

13. Vorrichtung nach Anspruch 1, bei der die Vorrichtung ausgestaltet ist, um unter Verwendung eines Endoskops oder invasiver Mittel in das Organ eingeführt zu werden.

## Revendications

1. Dispositif pour une insertion dans un organe d'un patient, comprenant :
un corps de capsule (12) ;
un ou plusieurs bras (10), qui sont fixés au corps de capsule et sont disposés pour se prolonger depuis le corps de la capsule dans l'organe de manière à entrer en contact avec une surface intérieure de l'organe et
à fixer le dispositif à l'emplacement souhaité ;
un résonateur (44) fixé au corps de capsule, qui est disposé pour faire vibrer le un ou plusieurs bras ;
un mécanisme de démontage (42), qui est disposé pour désengager le un ou plusieurs bras du corps de la capsule de manière à permettre au corps de capsule et au un ou plusieurs bras d'être éliminés ; et
un contrôleur (36), qui est placé de manière à activer et à désactiver ledit résonateur,
où, quand ils sont étendus, le un ou plusieurs bras sont disposés pour appliquer au moins une stimulation mécanique, tactile et nociceptive à des points de contact respectifs dans l'organe et le un ou plusieurs bras sont disposés pour transférer une vibration mécanique aux points de contacts respectifs.

2. Dispositif selon la revendication 1, où le corps de capsule est disposé de manière à être avalé par le patient, et où le un ou plusieurs bras sont disposés pour se prolonger depuis le corps de capsule vers l'estomac.

3. Dispositif selon la revendication 1 ou la revendication 2, où le un ou plusieurs bras comprennent au moins un matériau métallique, plastique et piézoélectrique.

4. Dispositif selon l'une quelconque des revendications 1 à 3, où le résonateur est disposé pour faire vibrer le un ou plusieurs bras avec une vibration aléatoire ayant des fréquences dans une largeur de bande étroite contenant des fréquences résonantes respectives de l'un ou plusieurs bras.

5. Dispositif selon l'une quelconque des revendications 1 à 4, où ledit contrôleur active ledit mécanisme de démontage de manière réceptive vers une condition de démontage, où la condition de démontage comprend au moins un calendrier prédéterminé, une condition de basse puissance d'une source de puissance du dispositif et une commande externe transmise au dispositif.

6. Dispositif selon la revendication 5, où le mécanisme de démontage comprend au moins un élément d'alliage à mémoire de forme (SMA), un moteur, un élément piézoélectrique, un solénoïde, un mécanisme de verrouillage à bille et une détente.

7. Dispositif selon l'une quelconque des revendications 1 à 6, et comprenant également une source de puissance (48) comprenant au moins un d'une batterie, d'une batterie rechargeable et d'une bobine de radio fréquence (RF) disposée pour accepter l'énergie RF transmise extérieurement.

8. Dispositif selon l'une quelconque des revendications 1 à 7, où le dispositif est disposé pour décharger une substance à l'emplacement souhaité dans l'organe.

9. Dispositif selon la revendication 8, et comprenant également un récipient maintenant la substance, prévu dans le corps de capsule.

10. Dispositif selon la revendication 9, où la substance est imprégnée dans au moins une d'une surface extérieure du corps de capsule et le un ou plusieurs bras.

11. Dispositif selon l'une quelconque des revendications 1 à 10, et comprenant également :
un transmetteur (50) ; et
une antenne (38) et un récepteur (40), qui sont disposés pour recevoir des commandes transmises vers le dispositif à partir d'un transmetteur externe dans une console externe (14) ; et
où ledit contrôleur (36) est disposé pour contrôler le fonctionnement du dispositif de manière réceptive sur les commandes et est également disposé pour transmettre par le transmetteur (50) des données fournies par le contrôleur à un récepteur externe dans la console (14).

12. Dispositif selon la revendication 1, où le corps de capsule et le un ou plusieurs bras sont disposés pour être insérés dans au moins un d'un vaisseau sanguin, une partie du tractus génito-urinaire, une partie du système nerveux central, un organe intra-abdominal, un organe thoracique et un membre du patient.

13. Dispositif selon la revendication 1, où le dispositif est disposé pour être inséré dans l'organe en utilisant un endoscope ou un moyen invasif.
